Europäisches Patentamt

European Patent Office

Office européen des brevets

(11) Publication number: **0 276 165 B1**

# EUROPEAN PATENT SPECIFICATION

(45) Date of publication of patent specification: **26.05.93**   (51) Int. Cl.5: **G01N 33/533**, G01N 33/543,
//G01N33/564,G01N33/577

(21) Application number: **88300527.4**

(22) Date of filing: **22.01.88**

(54) **Targetted liposomes and their use in immunoassay.**

(30) Priority: **23.01.87 GB 8701484**

(43) Date of publication of application:
**27.07.88 Bulletin  88/30**

(45) Publication of the grant of the patent:
**26.05.93 Bulletin  93/21**

(84) Designated Contracting States:
**AT BE CH DE FR IT LI NL SE**

(56) References cited:
**EP−A− 0 196 880**
**EP−A− 0 201 211**
**US−A− 4 550 086**
**US−A− 4 564 599**
**US−A− 4 610 869**

**NATURE, vol. 288, 1980; pp. 602−604***

(73) Proprietor: **UNIVERSITY COLLEGE LONDON**
**Gower Street**
**London WC1E 6BT(GB)**

(72) Inventor: **Huehns, Ernst Reinhard**
**18 Crundale Avenue**
**London, NW9 9PL(GB)**
Inventor: **Gray, Atherton Gerald**
**8 Banbury Road**
**London, E9 7EB(GB)**

(74) Representative: **Collier, Jeremy Austin Grey et al**
**J.A.Kemp & Co. 14, South Square Gray's Inn**
**London WC1R 5EU (GB)**

Rank Xerox (UK) Business Services
(3.10/3.6/3.3.1)

EP 0 276 165 B1

## Description

THIS INVENTION relates to methods of immunoassay and more particularly to targetted liposomes useful in such methods.

The assay of cell − bound antigens and especially cell surface antigens makes it possible to detect, for example, changes in cell surface proteins or the presence of abnormal cells, which may be indicative of a specific pathological condition including, for example, autoimmune diseases such as haemolytic disease of the newborn or immune thrombocytopenia.

It is known to assay cell surface antigens by immunoflourescent labelling. For this purpose a fluorescent label has been conjugated to an antibody. In direct fluorescent labelling, an antibody specific to the cell surface antigen is used and this antibody is itself attached to the fluorescent label. In indirect fluorescent labelling an unlabelled immunoglobulin (especially a monoclonal antibody) specific to the cell surface antigen is used, and the fluorescent label is bound to an anti − immunoglobulin specific to the unlabelled immunoglobulin. The indirect method allows some enhancement of the signal because more than one fluorescent anti − immunoglobulin antibody is able to attach to each cell − bound immunoglobulin molecule. However, it is usually only possible to attach a few fluorescent molecules to each anti − immunoglobulin molecule. For example, with an IgG anti − immunoglobulin, it is only possible to attach four molecules of the label fluorescein isothiocyanate (FITC).

Liposomes have been used in the assay of cell − bound antigens, for example by binding a label to liposomes and subsequently attaching the labelled liposomes to an antibody which is specific to the antigen to be detected or measured. The label used may be, for example, a radioisotope or a fluorescent compound. The amount of label that may be associated with each liposome, and hence with each antigen to be detected, is greater than may be achieved when liposomes are not used and the label is attached directly to antibody in the ways described above. This is especially advantageous in the case of fluorescent labelling, where the degree of sensitivity achievable is limited by the number of fluorophore molecules that can be attached to one antibody.

In one published account of the use of labelled liposomes, the label is a fluorescent compound (J. Immunol methods 46 (1981) 141 − 151) and the antibody bound to the liposomes is a monoclonal antibody specific to a particular mouse antigen (H − 2$^k$). In this case the labelling achieved is stated to be 4 − 6 times greater than with standard immunofluorescent labelling. However, although this method achieves high sensitivity, it is highly specific since the labelled liposomes can attach themselves only to mouse cells of the H − 2$^k$ type.

In a second published account, (Nature 288 December 11 1980 p.602 − 604) protein A is attached to fluorescent liposomes so that the latter are targeted specifically at human cells previously incubated with anti − human $\beta_2$ − microglobulin antibody, but here again the specificity is high since protein A only binds to particular classes of antibodies.

EP − A − 196 880 discloses sacs, in particular vesicles, incorporating a detectable marker and use thereof in an assay for an analyte. Fluorescently labelled liposomes are described, however the concentra − tion of fluorescent compound contained in the liposomes is self − quenching, i.e. the fluorescent compound must be released from the liposome in order to be detectable.

EP − A − 201 211 describes the use of luminescent polymer microspheres for the detection of analytes in biological fluids and other samples. There is no disclosure of the use of liposomes for this purpose.

Thus a given batch of liposomes bearing a fluorescent label coupled to a targeting agent such as protein A or monoclonal antibody has only limited utility since the analytes that can potentially be detected or measured are restricted. It is therefore desirable to develop a method of labelling having greater generality, but no less sensitivity, so that one batch of targeted liposomes carrying a label can be used to assay a wide variety of analytes. The present invention provides a novel reagent which realizes this objective.

The present invention provides a biological reagent comprising a liposome containing a fluorescent compound and a second binding agent (as hereinafter defined) covalently attached to the surface of the liposome in which the liposome contains the fluorescent compound at the concentration that affords maximum fluorescence to the reagent when the compound is retained inside the liposome. This biological reagent may be used as a labelling device on a system which consists of (1) a support with a biological material bound thereto, (2) a first binding agent (as hereinafter defined) and (3) the biological reagent of the present invention in which the second binding agent (as hereinafter defined) has specific affinity for the first binding agent.

As used herein the term "first binding agent" means a species which can react specifically with both the support − bound biological material and the second binding agent as hereinafter defined. The term "second binding agent" as used herein means a species which attaches the label led liposomes to the complex of the biological material plus first binding agent by coupling specifically with the first binding agent, and not with the biological material itself. The biological reagent is hence specific to the first binding agent while the latter is specific to the support − bound biological material. The amount of label that is attached to the support therefore depends upon the amount of biological material present end on the amount of first binding agent. Whichever of these two is present in lesser amount determines the amount of label which becomes fixed to the support. Hence, depending on the conditions of its use, the reagent of the invention may be used to assay either the support − bound biological material or the said first binding agent. The present invention is especially useful in relation to the assay of the support − bound biological material itself which may be, for example, a cell − surface antigen. In appropriate cases, however, it can also be used to assay the first binding agent, for example in autoimmune disease where antibodies that spontaneously attach themselves to cell − surface antigens are the species to be assayed.

Although the description which follows relates largely to preferred embodiments where the support − bound biological material is the analyte, it will be understood that essentially the same technique may be applied to cases where the analyte is the first binding agent.

In a preferred embodiment of the invention the first binding agent is a mouse monoclonal antibody, specific for the cell − bound antigen which is the analyte to be assayed. The second binding agent, which is bound covalently to liposomes preferably containing carboxyfluorescein or a similar fluorescent compound, is then an antibody, from e.g. a sheep or goat, specific to mouse immunoglobulin.

The present invention provides a means for increasing the number of fluorescent molecules which can be associated with each immunoglobulin (Ig) molecule and it thus has excellent sensitivity. Liposomes containing a fluorescent compound produced in accordance with the invention are highly fluorescent and retain both their fluorescence and antibody − targetting after several months storage. Although this invention is described herein with particular reference to the use of fluorsecein as a label, liposomes can also incorporate other fluorophores, e.g. phycoerythrin.

A variety of lipids may be used to make the liposomes but account should be taken of features such as the degree of saturation, the transition temperature and the fatty acyl chain length. The inclusion of cholesterol in the fat is desirable to reduce membrane permeability. It is also necessary to include a suitable lipid to act as anchor for the covalent attachment of the second binding agent.

Any anchor lipid present in the liposomes should have similar properties to those of the structural lipid and have also a reactive group for protein coupling. Phosphatidyl ethanolamine (P.E.), for example, has a terminal amino group which can be substituted with the heterobifunctional agent N − hydroxysuccinimidyl 3 − (2 − pyridyldithio) propionate (SPDP) to give PE 3 − (2 − pyridyldithio) propionate (PE − DTP). This reac − tion is carried out before the liposomes are formed.

In general, lipids are preferred which are neutral, fully saturated and have a melting point above 37°C. Preferred lipids and the preferred relative proportions of them which have been found to be useful are as follows: dipalmitoyl phosphatidylcholine (DPPC) 66%; cholesterol 33%; dipalmitoyl phosphatidylethanolamine − DTP (DPPE − DTP) 1%.

The liposomes may be made in known manner, e.g. as follows. The lipids are dissolved in a chloroform − methanol mixture and cast on to the wall of a round − bottomed flask by rotary evaporation. Solvent traces are removed by freeze − drying. An aqueous solution of a water − soluble fluorescent dye is then added. The concentration of the dye may vary within wide limits but approximately 20 mmolar carboxyfluorescein (CF) as the sodium salt gives maximum fluorescence for that compound. The lipids are hydrated with the aqueous component by vortexing until all the lipid has been taken up from the walls of the vessel. The suspension is then transferred to the cabinet of a probe sonicator where it is maintained a few degrees above the transition temperature of the lipid. Sonication is continued for approximately 30 minutes after optical clearing of the suspension. The liposome dispersion is centrifuged at 1000g to remove fragments from the tungsten probe and may be left for a few hours to anneal. Unencapsulated CF is removed by filtration on a small column packed with Sephadex® G50 and the liposomes are then ready for coupling to the antibody.

Purified antibody is reacted with the same cross − linking agent (SPDP) as the anchor lipid and then activated by mild reduction with dithiothreitol. The reactants are separated at each stage by gel filtration. The activated antibody is immediately mixed with the liposomes and allowed to couple at room temperature for 24 hours. Uncoupled protein is removed by gel filtration, and the liposome − antibody complexes are sterilised by passage through a 0.45µ filter and stored away from light at 4°C.

This method results in the synthesis of small unilamellar vesicles (SUV) which are more stable than other forms of liposomes such as multilamellar vesicles (MLV) or liposomes made by the reverse − phase method (REV). These other forms of liposomes have a higher encapsulated volume than SUV and may be useful for special applications. However, the method of determining the maximally fluorescent concentration of fluorophore and of attaching the anti−immunoglobulin antibody to the liposome is the same.

The method of the present invention of assaying a support−bound biological material or a first binding agent (as hereinbefore defined) complementary thereto comprises incubating a conjugate of the said biological material and first binding agent with an excess of the biological reagent comprising a liposome into which a fluorescent label has been incorporated as described above and a second binding agent (as hereinbefore defined) covalently attached to the liposome; separating the unbound excess of the said reagent from the conjugate having liposomes bound thereto; and finally observing the bound and/or the unbound label in the intact liposome.

In a preferred embodiment the biological material comprises cell surface antigens on lymphocytes and the first binding agent is mouse monoclonal antibody to the lymphocytes. The biological reagent may then comprise sheet anti−mouse antibody covalently coupled to liposomes containing a water soluble compound of carboxyfluorescein as the fluorophore.

The wide applicability of the method of the present invention is shown by another preferred embodiment wherein the support bound biological material comprises DNA or RNA fragments which, following gel electrophoresis, have been 'blot' transferred to nitrocellulose or a chemically reactive paper. In this embodiment the first binding agent is a DNA or RNA probe covalently linked to biotin or avidin. Following incubation of this agent with the analyte the biological reagent of the invention is added, the latter consisting of avidin or biotin respectively as the second binding agent attached to fluorescent liposomes. After removal of excess of the biological reagent, the presence of the analyte is disclosed by a band of fluorescence in the gel electrophoresis pattern.

Specific polypeptides may also be detected after gel electrophoresis by this method; they are blotted onto nitrocellulose, and then incubated, first with an antibody which has biotin or avidin attached thereto. Avidin or biotin−bearing fluorescent liposomes respectively are then added and the polypeptides are revealed by fluorescence following removal of excess of the biological reagent.

A drawback inherent in the use of fluorescent−labelled liposomes as biological reagents is fluorescence quenching, which can severely limit the degree of sensitivity of the reagent.

The biological reagent of the present invention is rendered optimally sensitive by using the particular concentration of fluorescent compound that affords maximum fluorescence to the reagent when the fluorophore is retained inside the liposome. The determination of this optimum concentration is described in Example 1 below. It has also been found that such amplification of the fluorescent signal does not effect a corresponding increase in the background fluorescence ("noise") level, as is shown in Example 2. This greater signal to noise ratio, taken in conjunction with the optimum fluorophore concentration as hereinbefore described, affords an advantage to the biological reagent of the present invention in assays where the analyte is either partly or fully undetectable by standard known methods. For example, in one preferred embodiment of the method of the present invention, involving the assay of cell−surface antigens, it has proved possible to demonstrate the presence of certain antigens that are not manifested by other fluorescent methods.

The Examples which follow illustrate certain aspects of the invention.

EXAMPLE 1

This example shows how the maximally fluorescent concentration of fluorophore is determined. Because of the effect of self−quenching, increasing the concentration of the fluorphore gives a peak value after which further increases cause a diminution of fluorescence. Thus for each fluorophore the optimum concentration may be determined by a family of liposomes identical in every respect apart from the concentration of entrapped fluorophore. Table I shows the fluorescence values of such a family containing various concentrations of the fluorophore carboxyfluorescein (CF):

## TABLE I

| Concentration of carboxyfluorescein in liposomes | Fluorescence units | |
|---|---|---|
| | Before addition of Triton | After addition of Triton |
| 1mM | 234 | 67 |
| 10mM | 253 | 264 |
| 20mM | 384 | 434 |
| 40mM | 114 | 454 |
| 100mM | 81 | 1508 |
| 200mM | 35 | 2104 |

An encapsulated concentration of circa 20mM CF is therefore selected for this fluorophore.

EXAMPLE 2

This example shows how a cell which is easily detectable by conventional staining can be made even brighter by the present invention. $2 \times 10^5$ peripheral blood lymphocytes were plated out in microtitre wells. 50$\mu$l of a 2.5$\mu$g/ml solution of UCHT1 monoclonal antibody specific for certain surface antigens present on lymphocytes was added for 30 minutes, after which the cells were washed three times in culture medium. 50$\mu$l of a saturated solution of FITC−labelled sheep anti−mouse immunoglobulin (S$\alpha$M−FITC) or 50$\mu$l of small unilamellar liposomes coupled with sheep anti−mouse immunoglobulin (S$\alpha$M−SUV) was added and the cells were incubated for 30 minutes after which they were washed three times. All incubations and washings were done at 4°C. The cells were transferred to small plastic tubes and studied by flow cytometry. The following results were obtained:

## TABLE II

| | % positive cells | Mean cell fluorescence |
|---|---|---|
| SαM-FITC (background) | 8 | 16 |
| cells + SαM-SUV (background) | 0.3 | 12 |
| cells + UCHT1 + SαM-FITC | 77 | 50 |
| cells + UCHT1 + SαM-SUV | 74 | 351 |

This shows a seven-fold increase in cell brightness of liposome-stained cells compared to FITC stained cells (351 v 50 MCF). This result was not achieved at the expense of background fluorescence (cells with only second layer added) as the latter was in fact reduced.

EXAMPLE 3

This example illustrates how a cell which is negative by conventional staining can be shown to be positive by the present invention.

Peripheral blood lymphocytes were stained as above with the exception that the monoclonal antibody which was used was DMS-2, an antibody to interleukin-2 (IL-2).

The results were as follows:

## TABLE III

| | % positive cells | Mean cell fluorescence |
|---|---|---|
| Cells + SαM-FITC (background) | 12 | 12 |
| Cells + SαM-SUV (background) | 9.5 | 8.3 |
| Cells + DMS-2 + SαM-FITC | 7.5 | Nil |
| Cells + DMS-2 + SαM-SUV | 29 | 15.8 |

Thus FITC stained cells were not measurably more fluorescent than the background fluorescence, whereas cells stained using the present invention show that some 20% of cells more than the background have IL-2 present on the cell membrane.

6

## EXAMPLE 4

This example illustrates how the background fluorescence of cells incubated with diluted liposomes compare with the autofluorescence of cells by themselves.

Peripheral blood lymphocytes ($2 \times 10^5$ per well) were incubated with SαM − SUV in various dilutions for 30 minutes then washed three times with medium before being analysed by flow cytometry.

The results obtained were as follows:

**TABLE IV**

|  | Mean cell fluorescence |
|---|---|
| cells alone | 14.5 |
| cells + SαM-SUV (serial dilutions of liposomes in buffer)  1:1 | 17.5 |
| 1:2 | 16.1 |
| 1:4 | 15.4 |
| 1:8 | 15.4 |
| 1:16 | 15.1 |
| 1:32 | 14.4 |

Thus at a dilution of liposomes between 1:16 and 1:32 the background fluorescence of the cells is equivalent to the autofluorescence. The following table (Table V) shows the results of the same liposome dilutions incubated with cells which have been labelled with first layer antibody, in this case UCHT1. For comparison cells were also incubated with an optimum concentration of SαM − FITC.

TABLE V

|  | % positive cells | Peak Channel number |
|---|---|---|
| cells + SαM-FITC | 76 | 131 |
| cells + SαM-SUV (serial dilutions of liposomes in buffer) |  |  |
| 1:1 | 71 | 241 |
| 1:2 | 73 | 244 |
| 1:4 | 75 | 217 |
| 1:8 | 75 | 210 |
| 1:16 | 71 | 184 |
| 1:32 | 66 | 164 |
| 1:64 | 71 | 151 |

The peak channel number indicates the median cell brightness with the cell distribution on a log scale. Thus at a liposome dilution of 1/32 where the background fluorescence is equivalent to autofluorescence, (see Table IV) the present invention is still superior to SαM − FITC in terms of cell brightness (164 v 131).

Fluorescent immunoliposomes made in the manner of the present invention may be used in the study of cell phenotyping using flow cytometry, fluorimetry and UV microscopy. For use with the latter it may be useful to co − incorporate an anti − bleaching agent such as sodium azide or paraphenylene diamine which permit prolonged observation and photography of fluorescent targets before fading. Immunoliposomes are stable in a 1% solution of formaldehyde which fixes cells. This enables labelled cells to be stored or batched before analysis without deterioration of the fluorescent signal. A batch of liposomes may be conveniently synthesized in 48 hours. A convenient quantity of 6 ml gives enough for at least 500 tests.

The present invention may be applied inter alia in the following situations:

(i) cell phenotyping. This includes: the detection of antigens on leukaemic blasts, malignant lymphoma cells or carcinoma cells which have been classified as undifferentiated and which are of uncertain origin; the screening of panels of monoclonal antibodies and the demonstration of positivity at a level of antigen density not hitherto possible; earlier detection of differentiation activation antigens during cell ontogeny and proliferation; double staining techniques where the signal from the fluorescein label requires to be amplified in order to give parity with another colour label; fluorescent labelling of tissues sections in the detection of malignant cells; reticulocyte counting using anti − transferrin or anti − transferring − receptor antibody as first layer.

Simple substitution of anti−human for anti−mouse antibody conjugated to the liposomes extends the application of the present invention to HLA A, B and Dr typing. It also extends the invention to the detection of antibodies that have attached to cell surface antigens in a disease process, for example immune or autoimmune disease such as haemolytic anaemia, immune thrombocytopenia and immune neutropenia; and to the detection of autoantibodies that exist on cells present in tissue sections.

(ii) detection of DNA restriction fragments, RNA and polypeptides after "blotting", as described above. This methods avoids the hazard and inconvenience of using a highly radioactive probe with limited shelf life.

(iii) fluorescent immunosorbent assay. This is the assay of antigen or antibody which has been immobilised by attachment to a solid surface e.g. a microtitre plate. As in the conventional enzyme linked immunosorbent assay (ELISA) the antigen under test is first bound to the solid phase followed by addition of the antibody under test. After washing, antibody bound to antigen is revealed by addition of immunofluorescent liposomes.

**Claims**

1.  A biological reagent comprising a liposome containing a fluorescent compound and a second binding agent covalently attached to the surface of the liposome, in which the liposome contains the fluorescent compound at the concentration that affords maximum fluorescence to the reagent when the compound is retained inside the liposome, and the second binding agent is a species which is capable of attaching the labelled liposome to a complex of a support−bound biological material and a first binding agent by reacting specifically with the first binding agent and not the biological material itself, the first binding agent being a species which reacts specifically with the biological material.

2.  A reagent according to claim 1 in which the fluorescent compound is a water−soluble salt of carboxyfluorescein.

3.  A reagent according to claim 1 or 2 in which the second binding agent is an antibody.

4.  A reagent according to claim 3 in which the antibody is sheep or goat anti−mouse immunoglobulin.

5.  A reagent according to claim 1 or 2 in which the second binding agent is avidin or biotin.

6.  A method of assaying a support−bound biological material or a first binding agent complementary thereto, the first binding agent being a species which reacts specifically with the biological material, which comprises incubating a conjugate of the said biological material and first binding agent with an excess of a biological reagent according to any of claims 1−5 in which the second binding agent is a species which attaches the labelled liposomes to the biological material by reacting specifically with the first binding agent and not the biological material itself, separating the unbound excess of the said reagent from the conjugate having liposomes bound thereto; and finally observing the bound and/or the unbound label in the intact liposome.

7.  A method according to claim 6 in which the biological material is an antigen and the first binding agent is a monoclonal antibody specific to the said antigen.

8.  A method according to claim 7 in which the second binding agent is an antibody to the said monoclonal antibody.

9.  A method according to any one of claims 6 to 8 in which the analyte is a cell surface antigen.

10. A method according to claim 6 in which the first binding agent comprises a specific reagent for the support−bound biological material and either biotin or avidin and the second binding agent comprises avidin or biotin respectively.

11. A method according to claim 10 in which the support−bound biological material comprises DNA or RNA fragments which have been 'blot' transferred and the said specific reagent is a DNA or RNA probe respectively.

EP 0 276 165 B1

**12.** A method according to claim 10 in which the support – bound biological material comprises polypep – tides which have been 'blot' transferred and the said specific reagent is an antibody to the said polypeptides.

**13.** A method according to claim 6 in which the conjugate of support – bound biological material and first binding agent is a conjugate of cell – bound antigen and auto – immune antibody.

**Patentansprüche**

**1.** Biologisches Reagens, umfassend ein Liposom, das eine fluoreszierende Verbindung enthält, und ein zweites Bindungsagens, das an die Oberfläche des Liposoms kovalent gebunden ist, und worin das Liposom die fluoreszierende Verbindung einer Konzentration enthält, die dem Reagens maximale Fluoreszenz verleiht, wenn die Verbindung innerhalb des Liposoms zurückgehalten wird, und das zweite Bindungsagens eine Spezies ist, die dazu fähig ist, das markierte Liposom an einen Komplex aus einem trägergebundenen biologischen Material und einem ersten Bindungsmittel zu binden, indem es spezifisch mit dem ersten Bindungsmittel und nicht mit dem biologischen Material selbst reagiert, wobei das erste Bindungsmittel eine Spezies ist, die spezifisch mit dem biologischen Material reagiert.

**2.** Reagens nach Anspruch 1, dadurch gekennzeichnet, daß die fluoreszierende Verbindung ein wasser – lösliches Salz von Carboxyfluorescein ist.

**3.** Reagens nach Anspruch 1 oder 2, dadurch gekennzeichnet, daß das zweite Bindungsmittel ein Antikörper ist.

**4.** Reagens nach Anspruch 3, dadurch gekennzeichnet, daß der Antikörper Schaf – oder Ziegen – Anti – Maus – Immunoglobulin ist.

**5.** Reagens nach Anspruch 1 oder 2, dadurch gekennzeichnet, daß das zweite Bindungsmittel Avidin oder Biotin ist.

**6.** Verfahren zur Analyse eines trägergebundenen biologischen Materials oder eines ersten dazu komple – mentären Bindungsmittels, wobei das erste Bindungsmittel eine Spezies ist, die spezifisch mit dem biologischen Material reagiert, umfassend: Inkubierung eines Konjugates des biologischen Materials und ersten Bindungsmittels mit einem Überschuß eines biologischen Reagens gemäß einem der Ansprüche 1 bis 5, in dem das zweite Bindungsmittel eine Spezies ist, die die markierten Liposome an das biologische Material bindet, indem es spezifisch mit dem ersten Bindungsmittel und nicht mit dem biologischen Material selbst reagiert, Abtrennung des ungebundenen Überschusses des Reagens aus dem Konjugat, das daran gebundene Liposome besitzt, und schließlich Bestimmung des gebundenen und/oder ungebundenen Markers im intakten Liposom.

**7.** Verfahren nach Anspruch 6, dadurch gekennzeichnet, daß das biologische Material ein Antigen ist und das erste Bindungsmittel ein monoklonaler Antikörper ist, der spezifisch für dieses Antigen ist.

**8.** Verfahren nach Anspruch 7, dadurch gekennzeichnet, daß das zweite Bindungsmittel ein Antikörper für den monoklonalen Antikörper ist.

**9.** Verfahren nach einem der Ansprüche 6 bis 8, dadurch gekennzeichnet, daß der Analyt ein Zellenoberflächen – Antigen ist.

**10.** Verfahren nach Anspruch 6, dadurch gekennzeichnet, daß das erste Bindungsmittel ein spezifisches Reagens für das trägergebundene biologische Material und entweder Biotin oder Avidin umfaßt, und das zweite Bindungsmittel Avidin bzw. Biotin umfaßt.

**11.** Verfahren nach Anspruch 10, dadurch gekennzeichnet, daß das trägergebundene biologische Material DNA – oder RNA – Fragmente umfaßt, die "Blot" – übertragen wurden und das spezifische Reagens eine DNA – bzw. RNA – Sonde ist.

**12.** Verfahren nach Anspruch 10, dadurch gekennzeichnet, daß das trägergebundene biologische Material Polypeptide umfaßt, die "Blot" – übertragen wurden und das spezifische Reagens ein Antikörper für diese Polypeptide ist.

**13.** Verfahren nach Anspruch 6, dadurch gekennzeichnet, daß das Konjugat des trägergebundenen biolo – gischen Materials und ersten Bindungsmittels ein Konjugat eines zellgebundenen Antigens und eines Autoimmun – Antikörpers ist.

**Revendications**

**1.** Réactif biologique comprenant un liposome contenant un composé fluorescent et un second liant fixé par covalence à la surface du liposome, dans lequel le liposome contient le composé fluorescent à la concentration permettant une fluorescence maximale du réactif lorsque le composé est retenu à l'intérieur du liposome et le second liant est une espèce capable de fixer le liposome marqué à un complexe formé par une substance biologique liée à un support et un premier liant, par réaction spécifique avec le premier liant et non avec la substance biologique même, le premier liant étant une espèce qui réagit spécifiquement avec la substance biologique.

**2.** Réactif selon la revendication 1, dans lequel le composé fluorescent est un sel hydrosoluble de carboxyfluorescéine.

**3.** Réactif selon la revendication 1 ou 2, dans lequel le second liant est un anticorps.

**4.** Réactif selon la revendication 3, dans lequel l'anticorps est une immunoglobuline de mouton ou de chèvre anti – souris.

**5.** Réactif selon la revendication 1 ou 2, dans lequel le second liant est de l'avidine ou de la biotine.

**6.** Procédé de dosage d'une substance biologique liée à un support ou d'un premier liant qui lui est complémentaire, le premier liant étant une espèce qui réagit spécifiquement avec la substance biologique, lequel comprend l'incubation d'un conjugué de ladite substance biologique et du premier liant avec un réactif biologique en excès selon l'une quelconque des revendications 1 à 5, dans lequel le second liant est une espèce qui fixe les liposomes marqués à la substance biologique par réaction spécifique avec le premier liant et non avec la substance biologique même ; la séparation dudit réactif en excès non lié d'avec le conjugué auquel sont liés les liposomes ; et enfin l'observation du marqueur lié et/ou du marqueur non lié dans le liposome intact.

**7.** Procédé selon la revendication 6, dans lequel la substance biologique est un antigène et le premier liant est un anticorps monoclonal spécifique dudit antigène.

**8.** Procédé selon la revendication 7, dans lequel le second liant est un anticorps dirigé contre ledit anticorps monoclonal.

**9.** Procédé selon l'une quelconque des revendications 6 à 8, dans lequel la substance à analyser est un antigène de surface cellulaire.

**10.** Procédé selon la revendication 6, dans lequel le premier liant comprend un réactif spécifique de la substance biologique liée à un support et soit de la biotine, soit de l'avidine et le second liant comprend respectivement de l'avidine ou de la biotine

**11.** Procédé selon la revendication 10, dans lequel la substance biologique liée au support comprend des fragments d'ADN ou d'ARN qui ont été transférés par "buvardage" et ledit réactif spécifique est une sonde respectivement d'ADN ou d'ARN.

**12.** Procédé selon la revendication 10, dans lequel la substance biologique liée à un support comprend des polypeptides qui ont été transférés par "buvardage" et ledit réactif spécifique est un anticorps dirigé contre lesdits polypeptides.

13. Procédé selon la revendication 6, dans lequel le conjugué composé de la substance biologique liée à un support et du premier liant est un conjugué composé d'un antigène lié à une cellule et d'un anticorps auto − immun.